# EUROPÄISCHE PATENTANMELDUNG

(11) **EP 0 745 585 A1**
(43) Veröffentlichungstag der Anmeldung: **04.12.1996**
(21) Anmeldenummer: 96108783.0
(22) Anmeldetag: 31.05.1996
(51) Int. Cl.: C07C 253/22, C07C 255/53, C07C 255/58

(54) **Verfahren zur Herstellung von Amino- und Hydroxybenzonitrilen**

(30) Priorität: 03.06.1995 DE 19520491
(71) Anmelder: SKW Trostberg Aktiengesellschaft, D-83308 Trostberg (DE)
(72) Erfinder: Erben, Hans Georg, Dr., Marietta, GA 30067-5715 (US); Graefe, Jürgen, Dr., 83308 Trostberg (DE); Moser, Wolfgang, 83308 Trostberg (DE); Wernthaler, Konrad, Dr., 83361 Kienberg (DE)
(74) Vertreter: Weiss, Wolfgang, Dipl.-Chem. Dr.

(57) **Zusammenfassung**

Mit dem Verfahren gemäß der vorliegenden Erfindung lassen sich Amino- oder Hydroxybenzoesäuren oder deren Ester mit Ammoniak an dotierten Borphosphat-Trägerkatalysatoren selektiv und in hohen Ausbeuten bei hoher Katalysator-Belastung zu Amino- bzw. Hydroxybenzonitrilen umsetzen

## Beschreibung

Gegenstand der vorliegenden Erfindung ist ein Verfahren zur Herstellung von Amino- oder Hydroxybenzonitrilen der allgemeinen Formel (I) 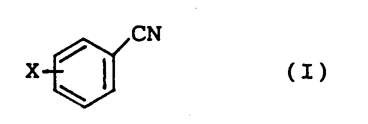 in der X für eine Amino- oder Hydroxy-Gruppe steht und der Phenylrest gegebenenfalls zusätzliche Substituenten trägt.

Für die Herstellung aromatischer Nitrile existieren zahlreiche unterschiedliche Verfahren, wobei im technischen Maßstab die Ammonoxidation von Methylarenen bevorzugt durchgeführt wird.

In Kirk-Othmer, "Encyclopedia of Chemical Technology", Vol. 15, S. 904 ff (1981) ist die Synthese von Benzonitril und Isophthalonitril durch Ammonoxidation von Toluol bzw. m-Xylol beschrieben. Mit diesem Verfahren lassen sich aber z.B. Kresole nicht in wirtschaftlicher Weise in Hydroxybenzonitrile umwandeln, da durch Oxidation gebildete Nebenprodukte den Katalysator bereits nach kurzer Zeit irreversibel inaktivieren. Hydroxybenzonitrile, die u. a. wichtige Zwischenstufen für die Synthese von Pflanzenschutzmitteln darstellen, sind somit auf diese Weise technisch nicht zugänglich.

Für die Herstellung von Hydroxybenzonitrilen eignet sich z.B. die Umsetzung von den Hydroxybenzoesäure-estern mit Ammoniak. So beschreibt die deutsche Offenlegungsschrift DE-OS 20 20 866 ein Verfahren, bei dem 4-Hydroxybenzonitril bei erhöhter Temperatur aus 4-Hydroxybenzoesäure-methylester hergestellt wird. Als Katalysator wird ein Phosphorsäure-Trägerkatalysator eingesetzt, der aus Phosphorsäure und dem Trägermaterial Kieselgel mit einer spezifischen Oberfläche von 100 m²/g bereitet wird und der eine Teilchengröße von 0,18 mm und einen Gehalt an Phosphorsäure von 65 % aufweist. Für eine Kontaktzeit am Katalysator von 20 Sekunden wird eine Ausbeute von 92 % angegeben. Gegen eine großtechnische Anwendung dieses Verfahrens spricht allerdings die geringe Katalysator-Belastung, die ein Maß für die Leistungsfähigkeit darstellt und angibt, welche Menge des Edukts pro Zeiteinheit und Menge des Katalysators (z.B. Mol · h⁻¹ · kg⁻¹) umgesetzt werden kann. Eine niedrige Katalysator-Belastung führt zwangsläufig zu unbefriedigenden Raum/Zeit-Ausbeuten und damit zu einer schlechten Wirtschaftlichkeit. Außerdem sind für die Herstellung des beschriebenen Katalysators relativ lange Temperzeiten von 20 bis 30 Stunden vorgesehen.

In der französischen Patentschrift FR 2 332 261 wird ebenfalls die Herstellung von 4-Hydroxybenzonitril durch Umsetzung eines 4-Hydroxybenzoesäure-esters mit Ammoniak in der Gasphase beschrieben. Als Katalysator wird Borphosphat verwendet, das an keinen Träger gebunden ist und somit wegen der geringen Abrieb-Beständigkeit des Borphosphats für den Einsatz in einer Wirbelschicht wenig geeignet ist.

Wirbelschicht-Verfahren besitzen gegenüber Festbett-Reaktionen den Vorteil, daß der Katalysator sehr viel weniger zu Verbackungen und Kanalbildungen neigt und deshalb die Standzeiten des Katalysators deutlich höher liegen.

Als nachteilig bei dem in der FR-PS 2 332 261 beschriebenen Verfahren ist auch anzusehen, daß die Reaktionsmischung aus dem 4-Hydroxybenzoesäure-ester und Ammoniak durch eine Schmelze des Esters geleitet wird. Unter diesen Bedingungen erfolgt z.T. eine Umwandlung des Edukts in Phenol, so daß verringerte Ausbeuten resultieren und ein 4-Hydroxybenzonitril erhalten wird, das durch beträchtliche Mengen an Phenol verunreinigt ist.

Ebenfalls mit der Herstellung von 4-Hydroxybenzonitril durch Umsetzung von Estern der 4-Hydroxybenzoesäure mit Ammoniak in der Gasphase befaßt sich das europäische Patent EP 0 074 116. Als Trägerkatalysator werden dabei nicht näher definiertes Silica, aber auch Aluminiumoxid verwendet; die Belegung erfolgt mit Borphosphat. Bei einer Kontaktzeit von 10 Sekunden und einer Reaktionstemperatur von 400 °C werden Ausbeuten von ca. 95 % erhalten. Auch bei diesem Verfahren ist jedoch die Katalysator-Belastung mit 0,54 mmol Ester pro Gramm Katalysator und Stunde verhältnismäßig gering.

Die sowjetische Patentschrift SU 1 007 717 offenbart einen speziellen Katalysator, der zur Herstellung von 4-Hydroxybenzonitril aus Estern der 4-Hydroxybenzoesäure und Ammoniak verwendet werden kann. Der tablettenförmige Katalysator setzt sich aus Bor(III)oxid, Vanadin(V)oxid und Phosphor(V)oxid zusammen, die zunächst einer wäßrigen Lösung aus Stärkekleister zugesetzt werden, dann im feuchten Zustand granuliert, verpresst und abschließend erhitzt werden. Mit Hilfe dieses Katalysators lassen sich jedoch keine zufriedenstellenden Durchsätze erzielen.

Aus den beschriebenen Nachteilen des Standes der Technik hat sich daher die Aufgabe gestellt, ein Verfahren zur Herstellung von Amino- und Hydroxybenzonitrilen zu entwickeln, bei dem Katalysatoren verwendet werden, die eine hohe Abriebsfestigkeit aufweisen und insbesondere auch bei Wirbelschicht-Verfahren verwendet werden können. Darüber hinaus sollen die Katalysatoren eine hohe Katalysator-Belastung aufweisen, damit eine hohe Raum/Zeit-Ausbeute erreicht und die Herstellung der genannten Nitrile in wirtschaftlicher Weise erfolgen kann. Des weiteren sollen sich diese Katalysatoren durch eine hohe Selektivität auszeichnen.

Gelöst wurde diese Aufgabe dadurch, daß man Amino- oder Hydroxybenzosäure-Derivate der allgemeinen Formel (II) 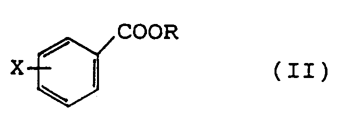 wobei R = H oder eine Alkylgruppe mit l bis 4 C-Atomen bedeutet und X für eine Amino- oder Hydroxy-Gruppe steht und wobei der Phenylrest gegebenenfalls zusätzliche Alkylsubstituenten, insbesondere C₁-C₄-Alkylsubstituenten trägt, bei Temperaturen zwischen 280 und 450°C über einen Borphosphat-Trägerkatalysator führt, der mit Übergangsmetall-Verbindungen der 5., 6., 12. oder 14. Gruppe des Periodensystems der Elemente oder Kombinationen davon dotiert ist und dessen spezifische Oberfläche mindestens 400 m²/g beträgt.

Überraschend hat sich dabei gezeigt, daß neben Amino- und Hydroxybenzoesäure-estern auch Amino- oder Hydroxybenzoesäuren selbst eingesetzt werden können, was nach den bislang beschriebenen Verfahren, bei denen ausschließlich Ester als Ausgangsstoffe eingesetzt wurden, nicht zu erwarten war.

Beim Verfahren entsprechend der vorliegenden Erfindung wird ein dotierter Borphosphat-Trägerkatalysator eingesetzt, wobei der Anteil des Borphosphats vorzugsweise 0,01 bis 15 Gew.-%, insbesondere 0,5 bis 5 Gew.-%, bezogen auf das Gewicht des Trägermaterials beträgt. Mit diesem Katalysator kann eine hohe Katalysatorbelastung von ≧ 1, vorzugsweise ≧ 2 mol Edukt pro kg Katalysator und h erreicht werden.

Gemäß einer bevorzugten Ausführungsform wird ein dotierter Borphosphat-Trägerkatalysator eingesetzt, der in der Weise hergestellt wurde, daß man in eine wäßrige Lösung von 0,01 bis 15 Gew.-%, vorzugsweise 0,5 bis 3 Gew.-% ,Phosphorsäure und 0,01 bis 15 Gew.-%, vorzugsweise 0,1 bis 3 Gew.-% Borsäure, 0,01 bis 5 Gew.-%, vorzugsweise 0,05 bis 2 Gew.-%, eines Salzes der Elemente Vanadium, Niob, Tantal, Chrom, Molybdän, Wolfram, Cadmium, Quecksilber, Germanium, Zinn, Blei oder Zink bzw. von beliebigen Mischungen dieser Salze löst und nachfolgend 1 Stunde bei Raumtemperatur rührt.

Vorzugsweise werden Salze eingesetzt, die als Kationen die jeweiligen Elemente und als Anionen Chlorid oder/und Sulfat enthalten. Es ist jedoch ohne weiteres möglich, daß die Salze als Anionen die jeweiligen Elemente enthalten (wie z.B. Vanadate, Permanganate, Molybdate oder Wolframate) und vorzugsweise als Kationen Ammonium-Ionen enthalten.

Anschließend werden zu dieser Salzlösung 20 bis 80 Gew.-% eines Trägermaterials, das vorzugsweise ausgewählt ist aus der Gruppe Siliciumdioxid, Kieselgel, Aluminiumoxid, Zirkoniumoxid oder Titanoxid bzw. Mischungen davon und eine spezifische Oberfläche von mindestens 400 m²/g aufweist, zugegeben. Das Wasser wird anschließend - ggf. unter Vakuum - verdampft und der Katalysator erfindungsgemäß maximal 3 Stunden bei Temperaturen zwischen 100 und 500°C unter Überleiten eines schwachen Luftstroms getrocknet. Als besonders geeignete Trocknungs-Temperatur hat sich 150°C erwiesen.

Gemäß vorliegender Erfindung ist der Übergangsmetall-dotierte Borphosphat-Trägerkatalysator durch eine Oberfläche gekennzeichnet, die ≧ 400 m²/g und bevorzugt > 500 m²/g ist. Dieser Katalysator weist einen bevorzugten Porendurchmesser von 0,4 bis 25 nm, insbesondere 0,5 bis 15 nm auf.

Die eigentliche Umsetzung erfolgt z.B. in einem beheizbaren Glasrohr mit Fritteneinsatz, das einen Durchmesser von ca. 5 cm besitzt und mit 50 ml des dotierten Borphosphat-Trägerkatalysators gefüllt ist. Durch das Reaktionsrohr wird auf 280 bis 450°C vorgeheizter Ammoniak geleitet und gleichzeitig mittels elektronisch geregelter Heizung die gewünschte Reaktionstemperatur eingestellt.

Die erfindungsgemäße Reaktionstemperatur liegt zwischen 280 und 450°C, vorzugsweise zwischen 300 bis 420°C. Besonders gute Ausbeuten sind im Temperaturbereich zwischen 310 und 400°C zu erreichen.

Als Amino- oder Hydroxybenzoesäure-derivate im Rahmen der vorliegenden Erfindung sind sowohl die Säuren als auch deren Ester zu verstehen, die nach Erreichen der gewünschten Reaktionstemperatur in flüssiger oder gelöster Form kontinuierlich in den Gasstrom eingebracht und verdampft werden, wobei im letztgenannten Fall auf inerte Lösemittel, z.B. aliphatische und aromatische Kohlenwasserstoffe, Ether oder Nitrile, zurückgegriffen werden kann. Besonders geeignet sind z.B. Toluol, Benzol oder Benzonitril.

Eine Lösung oder Schmelze der Amino- oder Hydroxybenzoesäure bzw. entsprechender Ester wird in den Ammoniak-Strom unterhalb der Fritte eindosiert und dabei verdampft. Der Gasstrom passiert dann das Wirbelbett von unten nach oben; der Durchmesser der kugelförmigen Katalysator-Partikel sollte dabei zwischen 0,2 und 2 mm liegen. In einem nachgeschalteten Abscheider wird das Produkt kondensiert, wobei Ammoniak zurückgeführt und/oder zurückgewonnen werden kann. Inertgase, z.B. Stickstoff, sind bei diesem Kreislauf-Prozeß nicht erforderlich, können aber verwendet werden.

Falls das Verfahren in einem Festbett durchgeführt werden soll, empfehlen sich Katalysator-Formkörper mit einem Durchmesser zwischen 2,O und 8,O mm.

Mit Hilfe des erfindungsgemäßen Verfahrens lassen sich Ausbeuten und Selektivitäten bis zu 100 % und Katalysator-Belastungen bis zu 5 mol Ausgangsprodukt pro kg Katalysator und Stunde erzielen.

Ein weiterer Gegenstand der vorliegenden Erfindung ist ein Borphosphat-Trägerkatalysator, der mit einer oder mehreren Übergangsmetall-Bindungen der 5., 6., 12. oder 14. Gruppe des Periodensystems der Elemente dotiert ist und dessen spezifische Oberfläche mindestens 400 m²/g beträgt. Dieser Katalysator kann zur Herstellung von aromatischen Amino- oder Hydroxysubstituierten Nitrilen durch Umsetzung von aromatischen Amino- oder Hydroxy-substituierten Carbonsäuren oder Carbonsäureestern mit Ammoniak verwendet werden.

Die nachfolgenden Beispiele sollen das erfindungsgemäße Verfahren veranschaulichen.

### Beispiele

### Herstellung des Katalysators

### Beispiel 1

5,88 g einer 85 %igen ortho-Phosphorsäure und 3,33 g einer 99,8 %igen Borsäure werden in 500 g dest. Wasser gelöst. Nachdem darin zusätzlich 3,6 g Zinksulfat gelöst sind, wird 1 Stunde bei Raumtemperatur gerührt. Anschließend werden 500 g Kieselgel zugegeben, dessen Oberfläche > 400 m²/g beträgt und das einen Porendurchmesser zwischen 0,4 und 25 nm aufweist. Anschließend wird bei 60°C unter vermindertem Druck bis zur Trockene eingedampft und abschließend 3 Stunden lang auf 150°C unter Überleiten eines schwachen Luftstroms erhitzt. Nach dem Abkühlen kann der so erhaltene Trägerkatalysator wie beschrieben eingesetzt werden.

Die Katalysatoren der Beispiele 2 bis 5 wurden analog Beispiel 1 hergestellt. Anstelle von 3,6 g Zinksulfat wurde mit folgenden Verbindungen dotiert:
- Beispiel 2:: 3,2 g Ammoniumperwolframat
- Beispiel 3:: 2,8 g Zinnchlorid
- Beispiel 4:: 0,6 g Ammoniummetavanadat
- Beispiel 5:: 1,4 g Zinnchlorid + 1,6 g Ammoniumperwolframat

### Verfahrensbeispiele

### Beispiel 6

Ein beheizbares Glasrohr (Durchmesser 5 cm) mit Frittenboden, wird mit 50 ml des nach Beispiel l hergestellten Zink-dotierten Borphosphat-Trägerkatalysators befüllt. Durch das Reaktionsrohr wird auf 340°C vorgeheizter Ammoniak geleitet und gleichzeitig mittels elektronisch geregelter Heizung eine Reaktionstemperatur von 340°C eingestellt. Sobald diese erreicht ist, werden 200 mmol 4-Hydroxybenzoesäure-methylester (4-HBSME) mit einer Geschwindigkeit von 50 mmol/h in den Gasraum unterhalb der Fritte dosiert. In einem Abscheider wird der ausgetretene Gasstrom abgekühlt, wobei sich das Reaktionsprodukt abscheidet. Auf diese Weise wird 4-Hydroxybenzonitril in 95 %-iger Ausbeute erhalten, als Nebenprokukt sind 0,6 % Phenol nachweisbar. Die Selektivität beträgt 99 % und die Katalysator-Belastung 2 mol · kg⁻¹ · h⁻¹.

In den nachfolgenden Beispielen (s. Tabelle) wird entsprechend dem Beispiel 6 verfahren.

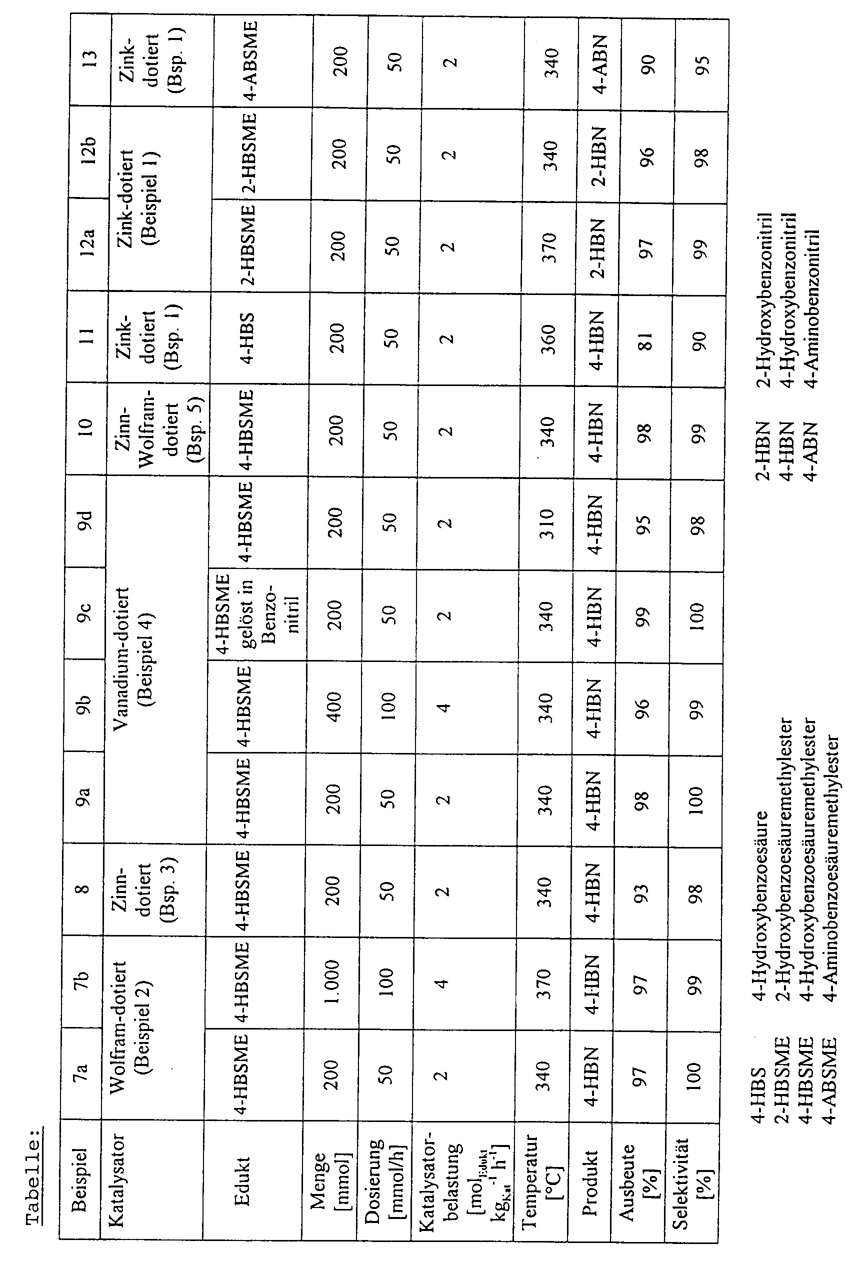

## Patentansprüche

1. Verfahren zur Herstellung von Amino- oder Hydroxybenzonitrilen der allgemeinen Formel (I) 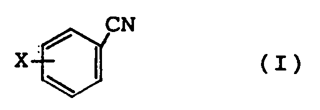 in der X für eine Amino- oder Hydroxy-Gruppe steht und der Phenylrest gegebenenfalls zusätzliche Alkylsubstituenten trägt, durch Umsetzung von Amino- oder Hydroxybenzoesäure-Derivaten der allgemeinen Formel (II) 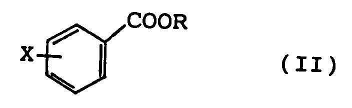 wobei R für H oder eine Alkylgruppe mit 1 bis 4 C-Atomen steht und der Phenylrest und X die oben genannten Bedeutungen besitzen, mit Ammoniak,
**dadurch gekennzeichnet,**
daß man die Umsetzung bei Temperaturen zwischen 280 und 450°C in Gegenwart eines Borphosphat-Trägerkatalysators durchführt, der mit Übergangsmetall-Verbindungen der 5., 6., 12. oder 14. Gruppe des Periodensystems der Elemente oder deren Kombinationen dotiert ist und dessen spezifische Oberfläche mindestens 400 m²/g beträgt.

2. Verfahren nach Anspruch 1,
**dadurch gekennzeichnet,**
daß der Anteil des Borphosphats 0,01 bis 15 Gew.-%, vorzugsweise 0,5 bis 5 Gew.-%, bezogen auf das Gewicht des Trägermaterials beträgt.

3. Verfahren nach Anspruch 1 oder 2,
**dadurch gekennzeichnet,**
daß man einen Borphosphat-Trägerkatalysator verwendet, der durch Behandlung des Trägermaterials mit wäßrigen Lösungen von 0,01 bis 15 Gew.-% Phosphorsäure, 0,01 bis 15 Gew.-% Borsäure und 0,01 bis 5 Gew.-% Salzen von Übergangsmetallen der 5., 6., 12. oder 14. Gruppe des Periodensystems der Elemente oder Kombinationen solcher Salze hergestellt wurde.

4. Verfahren nach einer der Ansprüche 1 bis 3,
**dadurch gekennzeichnet,**
daß das Trägermaterial aus der Gruppe bestehend aus Siliciumdioxid, Kieselgel, Aluminiumoxid, Titanoxid oder Zirkoniumoxid bzw. Mischungen davon ausgewählt wird.

5. Verfahren nach einem der Ansprüche 1 bis 4,
**dadurch gekennzeichnet,**
daß als Übergangsmetall-Verbindungen Salze der Elemente Vanadium, Niob, Tantal, Chrom, Molybdän, Wolfram, Cadmium, Quecksilber, Germanium, Zinn, Blei oder Zink, bzw. Kombinationen dieser Salze eingesetzt werden.

6. Verfahren nach Anspruch 5,
**dadurch gekennzeichnet,**
daß die Salze als Kationen das jeweilige Element und als Anionen Chlorid oder/und Sulfat enthalten.

7. Verfahren nach Anspruch 5,
**dadurch gekennzeichnet,**
daß die Salze als Anionen das jeweilige Element und Ammonium als Kation enthalten.

8. Verfahren nach einem der Ansprüche 1 bis 7,
**dadurch gekennzeichnet,**
daß die spezifische Oberfläche des dotierten Borphosphat-Trägerkatalysators > 500 m²/g beträgt.

9. Verfahren nach einem der Ansprüche 1 bis 8,
**dadurch gekennzeichnet,**
daß der dotierte Borphosphat-Trägerkatalysator einen Porendurchmesser zwischen 0,4 und 25 nm, insbesondere zwischen 0,5 und 15 nm, besitzt.

10. Verfahren nach einem der Ansprüche 1 bis 9,
**dadurch gekennzeichnet,**
daß der dotierte Borphosphat-Trägerkatalysator bis zu 3 Stunden bei Temperaturen zwischen 100 und 500°C getrocknet wurde.

11. Verfahren nach einem der Ansprüche 1 bis 10,
**dadurch gekennzeichnet,**
daß die Amino- oder Hydroxybenzoesäure-Derivate in flüssiger oder in gelöster Form eingesetzt werden.

12. Verfahren nach einem der Ansprüche 1 bis 11,
**dadurch gekennzeichnet,**
daß die Benzoesäure-Derivate bei Temperaturen zwischen 300 und 420°C umgesetzt werden.

13. Verfahren nach einem der Ansprüche 1 bis 12,
**dadurch gekennzeichnet,**
daß nicht umgesetzter Ammoniak zurückgeführt und/oder zurückgewonnen wird.

14. Borphosphat-Trägerkatalysator, der mit einer oder mehreren Übergangsmetall-Verbindungen der 5., 6., 12. oder 14. Gruppe des Periodensystems der Elemente dotiert ist und dessen spezifische Oberfläche mindestens 400 m²/g beträgt.

15. Verwendung des Borphosphat-Trägerkatalysators nach Anspruch 14 zur Herstellung von aromatischen Amino- oder Hydroxy-substituierten Nitrilen durch Umsetzung von aromatischen Amino- oder Hydroxy-substituierten Carbonsäuren oder Carbonsäureestern mit Ammoniak.
